Europäisches Patentamt

(19) European Patent Office

Office européen des brevets

(11) Publication number: **0 003 329**
**B1**

(12) **EUROPEAN PATENT SPECIFICATION**

(45) Date of publication of patent specification: **23.09.81**

(21) Application number: **79100153.0**

(22) Date of filing: **19.01.79**

(51) Int. Cl.³: **C 07 D 501/12,**
**C 07 D 501/22**

(54) **Cephalexin-amide complex, process for its production and use in cephalexin-monohydrate production.**

(30) Priority: **01.02.78 AT 671/78**

(43) Date of publication of application:
**08.08.79 Bulletin 79/16**

(45) Publication of the grant of the European patent:
**23.09.81 Bulletin 81/38**

(84) Designated Contracting States:
**BE CH DE FR GB IT LU NL SE**

(56) References cited:
**US - A - 3 957 773**
**US - A - 3 781 282**

(73) Proprietor: **BIOCHEMIE Gesellschaft m.b.H.**
**A-6250 Kundl Tyrol (AT)**

(72) Inventor: **Ascher, Gerd, Dr.**
**Bahnhofstrasse 18**
**A-6300 Wörgl (AT)**
Inventor: **Nachtmann, Friedrich, Dr.**
**Anton-Renk-Strasse 4**
**A-6330 Kufstein (AT)**

(74) Representative: **Streitzig, Heinz, Dr. et al,**
**SANDOZ AG Patentabteilung**
**CH-4002 Basle (CH)**

Courier Press, Leamington Spa, England.

Cephalexin-amide complex, process for its production and use in cephalexin-monohydrate production

This invention provides a novel complex of Cephalexin [7 - β - (D - α - aminophenyl-acetamido) - 3 - methyl - ceph - 3 - em - 4 - carboxylic acid] and formamide. The complex in crystalline form has a weight content of 72% of Cephalexin and 28% of formamide. It is stable at room temperature and may be dried to a constant weight at 30° to 50°C.

In accordance with the invention, the Cephalexin - formamide complex may be produced by treatment of Cephalexin with formamide and, in a preferred embodiment, the Cephalexin is produced in known manner by silylating 7 - β - amino - 3 - methyl - ceph - 3 - em - 4 - carboxylic acid (7 - ADCA), and acylating the resulting silyl derivative.

The process may, for example, be carried out by silylating 7-ADCA in known manner, employing such silylating agents as trichloro-methylsilane, hexamethyldisilazane, dichlorodimethylsilane, triphenylchlorosilane, hexa-phenyldisilazane, N-trimethylsilyl-acetamide, N, O - bis - trimethylsilylacetamide or bis-tri-methylsilylurea. Preferably, the silylation is effected with hexamethyldisilazane in methylene chloride. In general, it is preferred to employ slightly more than 1 equivalent of the silylating agent (based on the amount of 7-ADCA) so that the O-monosilylated 7-ADCA derivative results.

The acylation step may also be effected in conventional manner employing, for example, D-phenylglycol chloride hydrochloride and carrying out the process in the presence of an HCl-acceptor, for example a heterocyclic compound containing a 5-membered heterocyclic ring, having 2 or 3 hetero atoms, at least one of which is nitrogen, the ring optionally being substituted by one or more alkyl groups. The process is suitably effected in a low-boiling, organic solvent.

Suitable heterocyclic HCl-acceptors include dialkylthiazoles, dialkylisoxazoles, and di-alkyloxazoles, e.g., 2,4-dimethylthiazole, 2,5 -dimethylthiazole, 4,5 - dimethylthiazole, 3,5 - dimethylisoxazole, 4,5 - dimethylisoxazole and 2,5 - dimethyloxazole, as well as 3,4,5 - tri-ethylisoxazole, 3,4 - dimethyl - 1,2,5 - oxadi-azole, 3,5 - dimethylpyrazole and 1,3,5 - tri-methylpyrazole. These materials have the advantage over HCl-acceptors preferred in the art, e.g. N,N - dimethylaniline and pyridine, of lower toxicity and better water-solubility.

After the acylation step, the reaction mixture is optionally filtered and organic solvent is suitably removed. The residue is then preferably taken up in a formamide/water mixture (preferably 95%/5% by volume). Upon adjusting the pH to 6.5 to 7.0 with a base, the Cephalexin-formamide complex crystallises out. Preferred bases include aqueous ammonia, triethylamine, tributylamine, sodium or potassium carbonate or dilute sodium hydroxide solution.

The Cephalexin-formamide complex of the invention is useful in the production and isolation of Cephalexin, in particular Cephalexin monohydrate.

Cephalexin is a valuable, orally administrable antibiotic, produced by acylation of 7-ADCA or a derivative thereof, e.g. a salt or an ester thereof, to introduce the D - α - aminophenylacetyl group. For this purpose, the amino group in D-phenylglycine derivative employed is customarily protected to avoid undesired side reactions. Suitable protecting groups include tert. butoxycarbonyl or 2,2,2 - trichloroethoxycarbonyl groups. However, the use of such protecting groups necessitates a further step after the acylation reaction to split off the protecting group. The protection of the amino group by protonation, i.e. by using for example D - phenylglycyl chloride in the form of an acid addition salt, in particular the hydrochloride, avoids the need for a subsequent disprotection step. However, it does lead to a series of disadvantages in the known methods for effecting the acylation, for example in an organic solvent and in the presence of an HCl-acceptor. For example, normally the reaction does not run to completion so that the Cephalexin product is contaminated with unreacted 7-ADCA and/or is obtained in lower yields. In addition, under these conditions, the phenylglycyl chloride hydrochloride, while protected to a large extent, nevertheless may take part in a series of side-reactions, for example conversion to phenylglycine or self-acylation to phenylglycylphenylglycine.

One proposed method for dealing with these problems involves conversion of the Cephalexin after the acylation step into suitable easily crystallisable complex or solvate from which the final product can be obtained in economical yields and pharmaceutically acceptable quality. For example, Austrian patent 314,731 (Eli Lilly) and W. German DOS 2,432,485 (Glaxo) describe a process for producing N,N - dimethylformamide and -acetamide complexes of Cephalexin. The antibiotic is then released for these complexes, by dissolution in mineral acid and adjustment to pH with a base, either as the monohydrate form or, after drying, as the water-free product. By this method, the Cephalexin is freed from 7-ADCA and other impurities.

These processes have the disadvantage, however, that they employ carcinogenic substances or substances convertible to carcinogens, in particular dimethylformamide or hexamethylphosphoric acid triamide. In the industrial production of orally adminstered antibiotics, it is advantageous to avoid the use of such materials and, in particular, compounds which convert to dimethylamine, which chemically or metabolically is easily activated to the highly dangerous carcinogen N - nitroso - dimethylamine.

In addition, these known processes employ, during the acylation step, toxic bases such as N,N - dimethylaniline and pyridine, which are difficult to remove from the end-product.

The present invention provides a process for the production and isolation of Cephalexin, not involving the use of toxic materials, namely a process for the production of Cephalexin monohydrate comprising the step of converting the Cephalexin resulting from the acylation step to the Cephalexin - formamide complex of the invention and converting this to Cephalexin monohydrate.

More particularly, the present invention provides processes for the production of Cephalexin monohydrate comprising

a) suspending a Cephalexin - formamide complex in water, for example at a temperature of or under 40°C, adding an appropriate amount of a mineral acid so that a solution is formed, adjusting the pH of the solution to 6.5 and, conveniently, seeding the solution, whereby the Cephalexin monohydrate crystallises out;

or b) dissolving a Cephalexin - formamide complex in a water-miscible organic carboxylic acid, e.g. acetic acid, at, for example, room temperature, diluting the solution with an excess, e.g. a three-fold excess, of its own volume of water, seeding the solution, and allowing the Cephalexin monohydrate to crystallise out, preferably at room temperature or below;

or c) mixing a Cephalexin-formamide complex with at least 50% (based on its own dry weight) of water but insufficient water to form a solution and stirring the mixture at a temperature of 50° to 60°C. The crystal suspension is thereby converted into the monohydrate which may be filtered off;

or d) dissolving a Cephalexin formamide complex, in an aqueous alkali, e.g. caustic soda or potash, ammonia or triethylamine, adjusting the pH of the solution to the isoelectric point of Cephalexin monohydrate by addition of acid, e.g. hydrochloric, sulphuric or phosphoric acid, whereby the Cephalexin monohydrate crystallises out.

The Cephalexin monohydrate in this manner possesses a high degree of purity, in particular it is far freer from 7-ADCA than, for example, the product obtained via the dimethylformamide complex of the prior art. Furthermore, the use of the formamide complex of the invention permits the avoidance, if desired, of the prior art process of dissolving in acid, heating the precipitation with a base.

Furthermore, process a) possesses the additional advantage over prior art processes that, because of the lower precipitation temperature, higher yields are obtained, while processes b), c) and d) are more economical and ecologically more acceptable than prior art processes.

It has also been found that the use, during the acylation step, of the heterocyclic bases described, results in a higher yield and quality of the formamide complex and, therefore the final monohydrate end-product.

The following Examples, in which all the temperatures are in °C, illustrate the invention.

### Example 1
#### Cephalexin-Formamide Complex
21.4 g of 7-ADCA are suspended in 200 ml of methylenechloride. 16.6 ml of Hexamethyldisilazane and 0.2 ml of trimethylchlorosilane are added and the mixture is refluxed for 24 hours. The resulting, practically clear, solution is cooled to +2° and 8 ml of a 0.65 N solution of 2,4 - dimethylthiazole hydrochloride in methylene chloride is added. To this mixture is added, in one portion, 13.56 g of 2,4 - dimethylthiazole and 20 g of D - phenylglycylchloride hydrochloride, whereupon the temperature rises to +5°. The mixture is stirred for 3 hours with ice cooling at 3—5° and a further 2 g of D - phenylglycylchloride hydrochloride are added. After a further 2 hours stirring at 3° a clear solution is obtained. The solvent is removed and the residue is taken up in 300 ml of 95% formamide. The pH is adjusted to 6.5 with aqueous ammonia ($\delta=0.8$). The solution is seeded with formamide complex and allowed to stand for 1 hour at room temperature and overnight in the refrigerator. The heading compound crystallises out and is filtered, washed with 50 ml of 95% formamide and twice, each time with 50 ml, of ethyl acetate. It is then dried at 40° to constant weight. M.p. 153—154°; $[\alpha]_{20}^{D} = 113.2$ (c = 0.5 in water).

### Example 2
#### Cephalexin-Formamide Complex
In manner analogous to that of Example 1, but employing 14.6 g of 3,5 - dimethylisoxazole as acid binding agent, the same product is obtained.

### Example 3
#### Cephalexin-Formamide Complex
In manner analogous to Example 1, but employing 11.5 g of 3,5 - dimethylpyrazole as acid binding agent, the same product is obtained.

### Example 4
#### Cephalexin-Monohydrate
20 g of Cephalexin-formamide complex obtained in any of Examples 1 to 3 is taken up at room temperature in 41 ml of dilute sulphuric acid (40 ml of water + 1 ml of concentrated sulphuric acid). The pH of the solution is adjusted by dropwise addition of further sulphuric acid to 2 and the resulting clear solution is warmed to 40°. The pH is adjusted to 4.4 by addition of ammonia at 40° and the heading compound crystallises out. The mixture is cooled, stirred at 0° for 1 hour and filtered to obtain the heading compound which is washed with 5 ml of 80% isopropanol and ether and dried at 50° *in vacuo.*
$[\alpha]_{20}^{D} = 149.5°$ (c = 0.5 in water).

### Example 5
*Cephalexin-Monohydrate*

20 g of the Cephalexin-formamide complex obtained in any one of Examples 1 to 3 is dissolved in 30 ml of glacial acetic acid and the clear solution is diluted with 90 ml of water. The solution is seeded with a crystal of Cephalexin monohydrate and allowed to crystallise at 0° overnight. The resulting heading compound is filtered off, washed with isopropanol and dried.
$[\alpha]_{20}^{D} = 149.5$ (c = 0.5 in water).

### Example 6
*Cephalexin-Monohydrate*

5 g of the Cephalexin-formamide complex obtained in any one of the Examples 1 to 3 are suspended in 20 ml of water, the suspension is heated to 60° and stirred at this temperature for 1 hour. During this time, the crystal suspension of the formamide complex is converted into the monohydrate. The mixture is allowed to cool and filtered to obtain the heading compound which is washed with isopropanol.

### Example 7
*Cephalexin-Monohydrate*

10 g of the Cephalexin-formamide complex, obtained in any one of the Examples 1 to 3, and an equivalent amount of 2N caustic soda is added portionwise to 20 ml of ice water, during which time the pH is maintained under 8.5. After dissolution, the solution is warmed to 50° and its pH is adjusted to 4.4 with 50% sulphuric acid to obtain the heading compound which crystallises out. The mixture is allowed to cool and the heading compound is filtered off, washed with isopropanol and dried.

## Claims

1. Cephalexin-formamide complex.

2. The complex of Claim 1, having, in crystalline form a weight content of 72% of Cephalexin and 28% of formamide.

3. Process for the production of the Cephalexin-formamide complex of Claim 1, which comprises treating Cephalexin with formamide and isolating the resulting complex.

4. Process of Claim 3, which comprises silylating 7 - ADCA (7 - beta - amino - 3 - methyl - ceph - 3 - em - 4 - carboxylic acid), acylating the resulting silyl derivative of 7 - ADCA with an appropriate reactive derivative of D - phenylglycine, treating the resulting Cephalexin with formamide, adjusting the pH of the resulting mixture to 6.5 to 7.0 and isolating the resulting Cephalexin-formamide complex.

5. The process of Claim 4, which comprises silylating 7 - ADCA, reacting the resulting silyl derivative of 7 - ADCA with D - phenylglycylchloride hydrochloride in the presence of an HCl-acceptor and in a low-boiling organic solvent, removing the solvent, taking up the resulting residue of Cephalexin in a formamidewater mixture, adjusting the pH of the resulting mixture to 6.5 to 7.0 by addition of a base, and isolating the resulting Cephalexin-formamide complex.

6. The process of Claim 5, in which the HCl-acceptor is a heterocyclic base containing a 5-membered heterocyclic ring having 2 or 3 hetero atoms at least one of which is nitrogen, and being unsubstituted or substituted by one or more alkyl groups, and in which a 95% by volume formamide/5% by volume water mixture is employed.

7. A process for the production of Cephalexin monohydrate, comprising

a) suspending Cephalexin - formamide complex in water, adding an appropriate amount of a mineral acid so that a solution is formed, and adjusting the pH of the solution to 6.5;

or b) dissolving Cephalexin-formamide complex in a water-miscible organic carboxylic acid, diluting the solution with an excess (of its own volume) of water, and seeding the solution;

or c) mixing Cephalexin-formamide complex with at least 50% (based on its own dry weight) of water but insufficient water to form a solution and stirring the mixture at a temperature of 50° to 60°C;

or d) dissolving Cephalexin-formamide complex in an aqueous alkali, and adjusting the pH of the solution to the isoelectric point of Cephalexin monohydrate by addition of acid.

## Revendications

1. Complexe céphalexine-formamide.

2. Le complexe de la revendication 1 ayant, sous forme cristalline, une teneur en poids de 72% de céphalexine et de 28% de formamide.

3. Procédé de préparation du complexe céphalexine - formamide de la revendication 1, comprenant le traitement de la céphalexine avec du formamide et l'isolement du complexe résultant.

4. Procédé selon la revendication 3, comprenant la silylation du 7 - ADCA (acide 7 - béta - amino - 3 - méthyl - céph - 3 - em - 4 - carboxylique), l'acylation du dérivé silylé du 7 - ADCA résultant avec un dérivé réactif approprié de la D - phénylglycine, le traitement de la céphalexine résultante avec du formamide, le réglage du pH du mélange obtenu entre 6,5 et 7,0 et l'isolement du complexe céphalexine-formamide résultant.

5. Le procédé de la revendication 4, comprenant la silylation du 7 - ADCA, la réaction du dérivé silylé du 7 - ADCA résultant avec le chlorhydrate du chlorure de D - phénylglycyle en présence d'un accepteur d'acide chlorhydrique et dans un solvant organique à bas point d'ébullition, l'élimination du solvant, la reprise du résidu de céphalexine résultant dans un mélange de formamide et d'eau, le réglage du pH du mélange résultant entre 6,5 et 7,0 par addition d'une base et l'isolement du complexe céphalexine-formamide résultant.

6. Le procédé de la revendication 5, dans lequel l'accepteur d'acide chlorhydrique est une base hétérocyclique contenant un hétérocycle à 5 chaînons comportant 2 ou 3 hétéroatomes dont l'un au moins est un atome d'azote, et étant non substitué ou substitué par un ou plusieurs groupes alkyle, et dans lequel on utilise un mélange de 95% en volume de formamide et de 5% en volume d'eau.

7. Un procédé pour la préparation du monohydrate de céphalexine comprenant

a) la mise en suspension du complexe céphalexine-formamide dans l'eau, l'addition d'une quantité appropriée d'un acide minéral de manière à former une solution, et le réglage du pH de la solution à 6,5; ou

b) la dissolution du complexe céphalexine-formamide dans un acide carboxylique organique miscible à l'eau, la dilution de la solution avec un excès (de son propre volume) d'eau, et l'ensemencement de la solution; ou

c) le mélange du complexe céphalexine-formamide avec au moins 50% (par rapport à son propre poids anhydre) d'eau mais avec une quantité d'eau insuffisante pour former une solution et l'agitation du mélange à une température de 50° à 60°C; ou

d) la dissolution du complexe céphalexine-formamide dans un alcali aqueux et le réglage du pH de la solution au point isoélectrique du monohydrate de céphalexine par addition d'acide.

**Patentansprüche**

1. Cephalexin-Formamidkomplex.

2. Der Komplex gemäss Anspruch 1, in kristalliner Form aus 72 gew. % Cephalexin und 28% Formamid bestehend.

3. Verfahren zur Herstellung eines Cephalexin-Formamidkomplexes gemäss Anspruch 1, beinhaltend die Behandlung von Cephalexin mit Formamid und die Isolierung des gebildeten Komplexes.

4. Verfahren gemäss Anspruch 3, worin 7-ADCS (7 - $\beta$ - Amino - 3 - methyl - ceph - 3 - em - 4 - carbonsäure) silyliert, das silylierte Derivat der 7-ADCS mit einem geeigneten reaktiven Derivat des D - Phenylglycins acyliert, das erhaltene Cephalexin mit Formamid behandelt, der pH-Wert des entstandenen Gemisches auf 6.5 bis 7.0 gebracht und der gebildete Cephalexin-Formamidkomplex isoliert wird.

5. Verfahren gemäss Anspruch 4, worin 7-ADCS silyliert, das erhaltene Silylderivat der 7-ADCS in Gegenwart eines HCl-Akzeptors und in einem bei niedriger Temperatur kochenden Lösungsmittel mit D - Phenylglycylchloridhydrochlorid umgesetzt, das Lösungsmittel entfernt, der Rückstand aus Cephalexin in ein Formamid-Wassergemisch aufgenommen, der pH-Wert des entstandenen Gemisches durch Zufügen einer Base auf 6.5 bis 7.0 gebracht und das gebildete Cephalexin-Formamidkomplex isoliert wird.

6. Verfahren gemäss Anspruch 5, worin der HCl-Akzeptor eine heterocyclische Base mit einem fünfgliedrigen hetereocyclischen Ring ist, welche 2 oder 3 Heteroatome enthält, von denen mindestens ein Stickstoff darstellt und unsubstituiert oder durch ein oder mehrere Akylgruppen substituiert ist und ein Gemisch aus 95 Vol. % Formamid und 5 Vol. % Wasser verwendet wird.

7. Verfahren zur Herstellung von Cephalexinmonohydrat, worin

a) der Cephalexin-Formamidkomplex in Wasser suspendiert, Mineralsäure bis zur Lösung zugefügt und der pH-Wert der Lösung auf 6.5 gebracht wird, oder

b) der Cephalexin-Formamidkomplex in eine mit Wasser mischbare organische Carbonsäure gelöst, die Lösung mit einem Ueberschuss (ihres eigenen Volumens) Wasser verdünnt und die Lösung geimpft wird, oder

c) der Cephalexin-Formamidkomplex mit mindestens 50% (seines eigenen Trockengewichtes), jedoch ungenügendem Wasser um eine Lösung zu bilden vermischt und das Gemisch bei einer Temperatur von 50 bis 60°C gerührt wird, oder

d) der Cephalexin-Formamidkomplex in eine wässrige Alkalibase gelöst, und die pH-Wert der Lösung mit Säure auf den isoelektrischen Punkt des Cephalexin-monohydrates gebracht wird.